# EUROPEAN PATENT SPECIFICATION

(11) **EP 1 754 491 B1**
(45) Date of publication and mention of the grant of the patent: **03.03.2010**
(21) Application number: 05743236.1
(22) Date of filing: 20.05.2005
(51) Int. Cl.: A61K 31/4178, A61K 45/00, A61K 9/06, A61K 9/70, A61K 31/439, A61K 47/14, A61P 25/02, A61P 27/02

(54) **OPHTHALMIC PERCUTANEOUSLY ABSORBED PREPARATION CONTAINING MUSCARINIC RECEPTOR AGONIST**
OPHTHALMOLOGISCHE PERKUTAN ABSORBIERBARE ZUBEREITUNG ENTHALTEND EINEN MUSCARIN-REZEPTOR-AGONIST
PRÉPARATION OPHTALMIQUE ABSORBÉE DE FAÇON PERCUTANÉE CONTENANT UN AGONISTE DU RÉCEPTEUR MUSCARINIQUE

(30) Priority: 21.05.2004 JP 2004151248
(43) Date of publication of application: 21.02.2007
(73) Proprietor: Senju Pharmaceutical Co., Ltd., Osaka-shi, Osaka 541-0046 (JP)
(72) Inventor: ISOWAKI, Akiharu, 6740061 (JP); OHTORI, Akira, Hyogo 6512273 (JP)
(74) Representative: von Kreisler, Alek
(86) International application number: PCT/JP2005/009709
(87) International publication number: WO 2005/113002

(56) References cited:
- EP-A- 1 221 315
- WO-A1-01/26648
- WO-A1-2004/064817
- JP-A- 08 509 716
- JP-A- 2003 531 168
- US-A- 5 869 086
- PETRONE D. ET AL: 'A double-blind, randomized, placebo-controlled study of cevimeline in Sjogren's syndrome patients with xerostomia and keratoconjunctivitis sicca.' ARTHRITIS & RHEUMATISM vol. 46, no. 3, 2002, pages 748 - 754, XP002997641
- VIVINO F.B. ET AL: 'Pilocarpine tablets for the treatment of dry mouth and dry eye symptoms in patients with Sjogren syndrome: a randomized, placebo-controlled, fixed-dose, multicenter trial' ARCHIVES OF INTERNAL MEDICINE vol. 159, no. 2, 1999, pages 174 - 181, XP002997642

## Description

### Technical Field

The present invention relates to a percutaneous absorption type ophthalmic preparation comprising a muscarinic receptor agonist, which is used for promoting lacrimal fluid secretion by administration to the skin surface of the eyelid. In addition, the present invention relates to a method of promoting lacrimal fluid secretion by administering a percutaneous absorption type ophthalmic preparation comprising a muscarinic receptor agonist to the skin surface of the eyelid.

### Background Art

Dry eye is a pathology caused by decreased amount of lacrimal fluid secretion and changes in the lacrimal fluid components, and may cause erosion of corneal and conjunctival epithelium, foreign body sensation and the like. Dry eye includes, for example, that caused by ophthalmic diseases such as Sjogren's syndrome, Stevens-Johnson syndrome, blepharitis, meibomitis, that caused by VDT (Visual Display Terminal) operation, use of contact lenses. As a method for the prophylaxis or treatment of dry eye, lacrimal fluid supplementation by administration of an artificial lacrimal fluid and instillation of eye drops containing a viscoelastic substance such as hyaluronic acid, chondroitin sulfate are usually practiced. However, the effect provided by these methods is temporary, and frequent administration is required. Furthermore, eye drops generally contain a preservative, which may cause side effects.

As one of the drugs that promote lacrimal fluid secretion, for example, muscarinic receptor agonists such as pilocarpine are known. Muscarinic receptor is also called a muscarinic acetylcholine receptor, is involved in neurotransmission and stimulation in the organs controlled by parasympathetic nerve, and is distinguished from nicotinic receptors in autonomic ganglion and ganglion junction (which are also called nicotinic acetylcholine receptors). In addition, muscarinic receptor is a 7-spanning transmembrane G protein conjugated receptor and divided into 5 subtypes of M1 to M5. Muscarinic receptor is widely distributed in the eye tissue, and is involved in various functions of lacrimal fluid secretion, changes in crystalline lens curvature, aqueous humor outflow dynamics. When a muscarinic receptor agonist is instilled, however, a side effect of miosis occurs, though lacrimal fluid is secreted.

In addition, treatment methods of dry eye which comprise administering a nicotinic acetylcholine receptor agonist (see WO-A-01/080844 and US-A-6,277,855), and compositions for promoting lacrimal fluid secretion, which comprises a component that activates PAR-2 (see JP 2001-181208 A and US publication No.2003203849) have been reported. These reports describe that these components can be used as a percutaneous absorption preparation.

As a percutaneous absorption preparation for treating ophthalmic diseases, for example, a percutaneous absorption ophthalmic adhesive agent comprising a drug intended to be delivered to the posterior eye including crystalline lens, vitreous body, choroid and retina and a drug-containing layer comprising a percutaneous absorption promoter in a base matrix has also been reported (see WO-A-01/26648 and EP-A-1221315).

Furthermore, a percutaneous absorption system for decreasing the intraocular pressure, which contains a pilocarpine base, which is a muscarinic receptor agonist, or a salt thereof has been reported (see JP 8-509716 A and US-A-5,869,086). However, this report does not describe promotion of lacrimal fluid secretion.

In other words, the above-mentioned references do not at all describe a lacrimal fluid promoting preparation comprising a muscarinic receptor agonist, which is of a percutaneous absorption type.

In addition, WO-A-04/064817 published on August 5, 2004 after filing of the base application of the present application reports a percutaneous absorption type preparation for treatment of ophthalmic diseases, which is adhered to the skin surface, including the anterior surface of the eyelid, to administer a therapeutic drug for ophthalmic diseases to the topical tissue of the eye, by skin permeation rather than via the systemic blood flow. However, this report does not describe a percutaneous absorption type preparation that promotes lacrimal fluid secretion, nor does it describe suppression of side effects such as miosis.

PETRONE, Dianne et al., A double-blind, randomized, placebo-controlled study of cevimeline in Sjogren's syndrome patients with xerostomia and keratoconjunctivitis sicca, Arthritis & Rheumatism, 2002, 46 (3), 748-754;

VIVINO, Frederick B. et al., Pilocarpine tablets for the treatment of dry mouth and dry eye symptoms in patients with Sjogren syndrome: a randomized, placebo-controlled, fixed-dose, multicenter trial, Archives of Internal Medicine, 1999, 159(2), 174-181
both disclose the oral use of a muscarinic receptor agonist, such as pilocarpine or cevimeline, for the treatment of dry eye by increasing the lacrimal flow.

US-A-5,869,086 disclose a transdermal therapeutic system comprising pilocarpine and isopropyl myristate for the treatment of glaucoma.

EP-A-1221315 disclose transdermal therapeutic systems comprising the percutaneous absorption enhancer isopropyl myristate for treating diseases of the posterior segment of the eye by administration to the skin of the eyelids.

### Disclosure of the Invention

The present invention aims at providing a percutaneous absorption type ophthalmic preparation, which can maintain a therapeutically effective concentration of a muscarinic receptor agonist that promotes lacrimal fluid secretion, and which is associated with a fewer side effects such as miosis.

Moreover, the present invention aims at providing a percutaneous absorption type ophthalmic preparation for administration to the skin surface of the eyelid, which is superior in a sustained lacrimal fluid secretion-promoting effect as compared to the administration to a region other than the skin surface of the eyelid, based on more substantial and direct transdermal transfer of the muscarinic receptor agonist into the topical tissue of the eye from the eyelid skin than by the transfer to the topical tissue of the eye via systemic circulation.

Another object of the present invention is to provide a method of promoting lacrimal fluid secretion, which comprises administering a percutaneous absorption type ophthalmic preparation comprising a muscarinic receptor agonist to the skin surface of the eyelid.

The present inventor has conducted intensive studies in an attempt to achieve the aforementioned objects and found that administration of a percutaneous absorption type ophthalmic preparation comprising a muscarinic receptor agonist to the skin surface of the eyelid enables sustained promotion of lacrimal fluid secretion without causing many side effects such as miosis and the like and, based on the findings, further developed the studies and completed the present invention.

Accordingly, the present invention provides the following.
(1) A percutaneous absorption type ophthalmic preparation comprising a muscarinic receptor agonist, to be administered to the skin surface of the eyelid for use in the promotion of lacrimal fluid secretion.
(2) The percutaneous absorption type ophthalmic preparation of the above-mentioned (1), wherein miosis is suppressed as compared to instillation.
(3) The percutaneous absorption type ophthalmic preparation of the above-mentioned (1) or (2), wherein the content of the muscarinic receptor agonist is 0.1-40 wt%.
(4) The percutaneous absorption type ophthalmic preparation of any of the above-mentioned (1)-(3), which further comprises an absorption promoter.
(5) The percutaneous absorption type ophthalmic preparation of the above-mentioned (4), wherein the content of the absorption promoter is 1-60 wt%.
(6) The percutaneous absorption type ophthalmic preparation of the above-mentioned (4) or (5), wherein the absorption promoter is isopropyl myristate.
(7) The percutaneous absorption type ophthalmic preparation of any of the above-mentioned (1)-(6), wherein the muscarinic receptor agonist is pilocarpine or cevimeline, or a pharmaceutically acceptable salt thereof.
(8) The percutaneous absorption type ophthalmic preparation of any of the above-mentioned (1)-(7), which is an adhesive agent.
(9) The percutaneous absorption type ophthalmic preparation of any of the above-mentioned (1)-(7), which is an ointment.
(10) The percutaneous absorption type ophthalmic preparation of any of the above-mentioned (1)-(7), which is a gel.
(11) A muscarinic receptor agonist to be administered to the skin surface of the eyelid for use in the promotion of lacrimal fluid secretion.
(12) The muscarinic receptor agonist of above-mentioned (11) and an absorption promotor.
(13) The above-mentioned (12), wherein the absorption promoter is isopropyl myristate.
(14) The above-mentioned (11)-(13), wherein the agent for promoting lacrimal fluid secretion is pilocarpine or cevimeline, or a pharmaceutically acceptable salt thereof.

### Brief Description of the Drawings

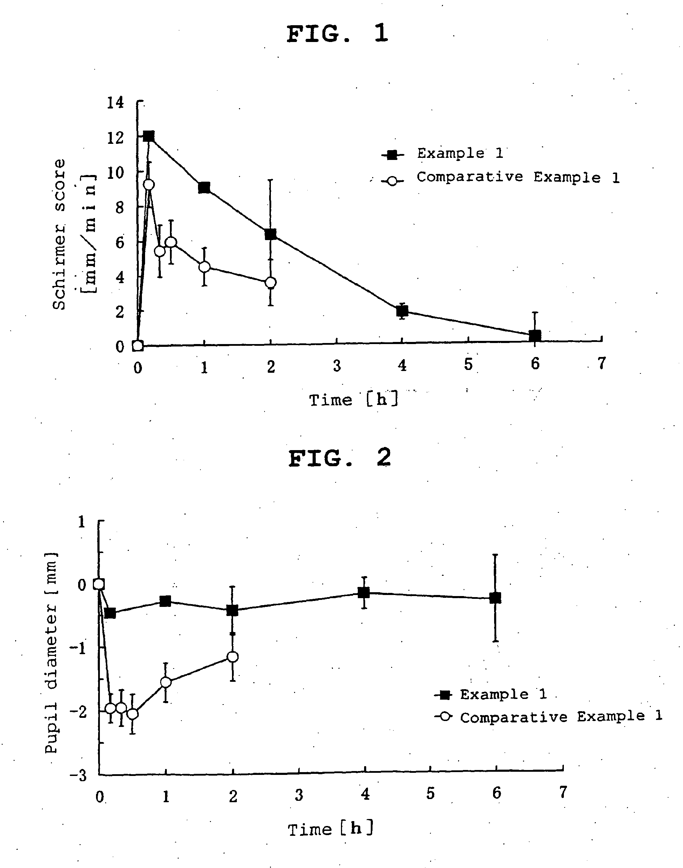

Fig. 1 is a graph showing increase in the amount of lacrimal fluid secretion by the administration of a pilocarpine hydrochloride-containing ointment (10 min after application n=1; 1 hr later n=2; and 2-6 hr later n=3), and a pilocarpine hydrochloride-containing eye drops (10 min-2 hr after instillation: n=8), in Experimental Example 1, wherein each value shows mean±standard error.
Fig. 2 is a graph showing changes in the pupil diameter by the administration of a pilocarpine hydrochloride-containing ointment (10 min after application n=1; 1 hr later n=2; and 2-6 hr later n=3), and pilocarpine hydrochloride-containing eye drops (10 min-2 hr after instillation: n=8), in Experimental Example 2, wherein each value shows mean±standard error.

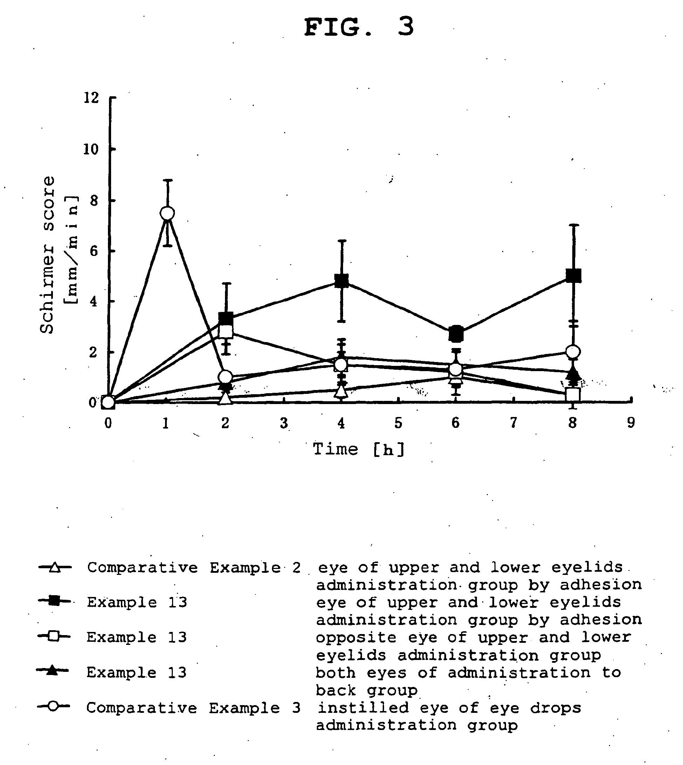

Fig. 3 is a graph showing increase in the amount of lacrimal fluid secretion of the eye (n=3) to which a pilocarpine hydrochloride-containing adhesive preparation (Example 13) was administered by adhesion to the skin of the upper and lower eyelids thereof, the eye (non-adhesion, n=3) opposite of the eye (n=3) to which a pilocarpine hydrochloride-containing adhesive preparation (Example 13) was administered by adhesion to the skin of the upper and lower eyelids thereof, both eyes (n=6) with administration of a pilocarpine hydrochloride-containing adhesive preparation (Example 13) by adhesion to the back, the eye (n=3) with administration of pilocarpine hydrochloride-containing eye drops (Comparative Example 3), and the eye (n=3) to which a base adhesive preparation (Comparative Example 2) was administered by adhesion to the skin of the upper and lower eyelids thereof, in Experimental Example 3, wherein each value shows mean±standard error.

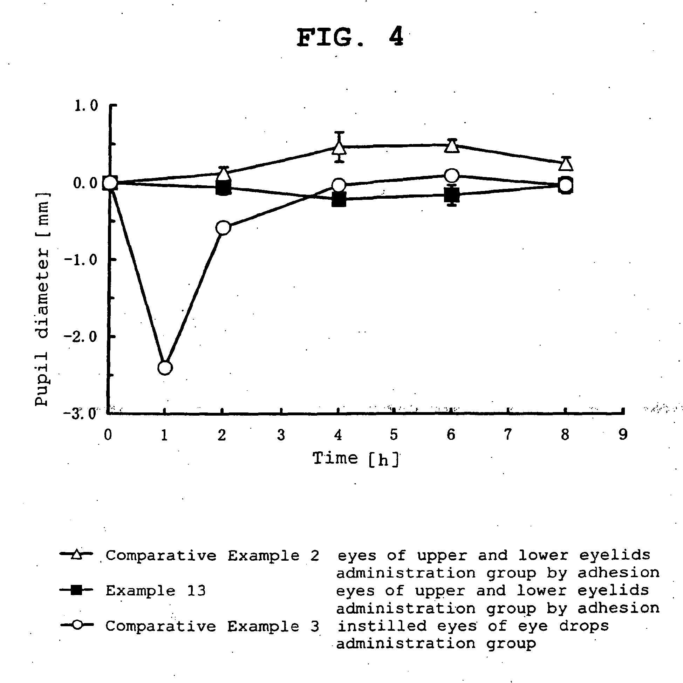

Fig. 4 is a graph showing changes in the pupil diameter of the eye (n=3) to which a pilocarpine hydrochloride-containing adhesive preparation (Example 13) was administered by adhesion to the skin of the upper and lower eyelids thereof, the eye (n=3) to which pilocarpine hydrochloride-containing eye drops (Comparative Example 3) were administered, and the eye (n=3) to which a base adhesive preparation (Comparative Example 2) was administered by adhesion to the skin of the upper and lower eyelids thereof, in Experimental Example 4, wherein each value shows mean±standard error.

### Best Mode for Embodying the Invention

The percutaneous absorption type ophthalmic preparation of the present invention may be any preparation as long as a muscarinic receptor agonist can be delivered to the topical tissue of the eye by administration to the skin surface including the eyelid and, for example, external preparations such as adhesive preparation, ointment, gel, cream and the like can be mentioned, with preference given to adhesive preparation, ointment and gel. In the present invention, an adhesive agent means a preparation that can be adhered to the skin, such as cataplasm, patch, tape, plaster and the like.

The percutaneous absorption type ophthalmic preparation of the present invention has superior lacrimal fluid secretion-promoting action. That is, the present invention provides a percutaneous absorption type agent for promoting lacrimal fluid secretion, which comprises a muscarinic receptor agonist.

In the present invention, the skin surface of the eyelids means that of the upper eyelid and the lower eyelid, as well as the skin surface of the vicinity thereof.

The percutaneous absorption type ophthalmic preparation of the present invention can promote sustained lacrimal fluid secretion by controlling the kind, amount and the like of the muscarinic receptor agonist to be contained in the preparation. In addition, the percutaneous absorption type ophthalmic preparation of the present invention permits easy control of the effective dose of the muscarinic receptor agonist for promoting lacrimal fluid secretion.

The present invention is described in more detail in the following.

The muscarinic receptor agonist to be used in the present invention may be any as long as it is capable of acting on a muscarinic receptor to activate a muscarinic receptor, and encompasses naturally occurring substances and artificially synthesized ones. For example, pilocarpine, cevimeline, carbachol, and muscarine, pharmaceutically acceptable salts thereof can be mentioned. Preferred are pilocarpine, cevimeline, and pharmaceutically acceptable salts thereof. As the pharmaceutically acceptable salt of pilocarpine, for example, salts with hydrochloric acid, sulfuric acid, nitric acid, acetic acid can be mentioned, with preference given to hydrochloride. Furthermore, as a pharmaceutically acceptable salt of cevimeline, for example, salts with hydrochloric acid, sulfuric acid, phosphoric acid, and tartaric acid can be mentioned, with preference given to hydrochloride.

The percutaneous absorption type ophthalmic preparation of the present invention can appropriately contain, where necessary, optional components generally used for the manufacture of pharmaceutical products, as long as the effect of the present invention is not impaired. For example, ointment base, gel base, base matrix of adhesive preparation, solvent, medicinal oil, surfactant, thickener, resin, absorption promoter, and moisturizer can be mentioned.

As the ointment base, for example, oleaginous base such as petrolatum, paraffin, plastibase, silicone, vegetable oil, lard, solders, and simple ointment; emulsion base such as hydrophilic ointment (vanishing cream), hydrophilic petrolatum, purified lanolin, water-absorbing ointment, hydrous lanolin, purified lanolin, and hydrophilic plastibase (cold cream) can be mentioned.

As the gel base, for example, thickening polymers such as carboxyvinyl polymer, polyacrylic acid, sodium polyacrylate, methylcellulose, polyvinyl alcohol, polyvinylpyrrolidone, polyethylene oxide, polyacrylamide, sodium alginate, gelatin, gum arabic, tragacanth rubber, guar gum, xanthan gum, and agar ; fatty acid esters such as isopropyl myristate, isopropyl palmitate, and propylene glycol oleate; fatty acids such as lactic acid, lauric acid, oleic acid, linoleic acid, and linolenic acid and the like; aliphatic alcohols such as lauryl alcohol, and oleyl alcohol; hydrocarbons such as squalene, can be mentioned.

As the solvent, for example, purified water, ethanol, lower alcohols, ethers, pyrrolidones, and ethyl acetate can be mentioned.

As the medicinal oil, volatile and nonvolatile oils, solvents and resins, which are generally used as external preparations for the skin, such as oil, can be mentioned, and any of liquid, paste and solid can be used at ambient temperature. For example, higher alcohols such as cetyl alcohol, isostearyl alcohol; fatty acids such as isostearic acid, and oleic acid; polyhydric alcohols such as glycerol, sorbitol, ethylene glycol, propylene glycol, and polyethylene glycol; esters such as myristyl myristate, hexyl laurate, decyl oleate, isopropyl myristate, and glycerol monostearate can be mentioned.

As the surfactant, for example, anionic surfactant, cationic surfactant, nonionic surfactant and amphoteric surfactant can be used.

As the anionic surfactant, for example, fatty acid salts, alkyl sulfates, polyoxyethylenealkyl sulfates, alkylsulfocarboxylates, and alkyl ether carboxylates can be mentioned.

As the cationic surfactant, for example, amine salts, quaternary ammonium salts and the like can be mentioned.

As the nonioinic surfactant, for example, polyoxyethylene hydrogenated caster oil, polyoxyethylene fatty acid esters, polyoxyethylene alkyl ethers, and polyoxyethylenesorbitan fatty acid esters can be mentioned.

As the amphoteric surfactant, for example, alkyl betaines, dimethylalkylglycines, and lecithin can be mentioned.

As the thickener and resin, for example, sodium polyacrylate, cellulose ether, calcium alginate, carboxyvinyl polymer, ethylene-acrylic acid copolymer, vinylpyrrolidone polymer, vinyl alcohol-vinyl pyrrolidone copolymer, nitrogen-substituted acrylamide polymer, polyacrylamide, cationic polymers (e.g., cationic guar gum), dimethylacryl ammonium polymer, acrylic acid-methacrylic acid acryl-copolymer, polyoxyethylene-polypropylene copolymer, polyvinyl alcohol, pullulan, agar, gelatin, tamarind seed polysaccharides, xanthan gum, carageenan, high-methoxylpectin, low-methoxylpectin, guar gum, gum arabic, crystalline cellulose, arabinogalactan, karaya gum, tragacanth gum, alginic acid, albumin, casein, cardlan, gellan gum, dextran, cellulose, polyethylene imine, high-polymerized polyethylene glycol, cationated silicone polymer, synthetic latex, acryl silicone, trimethylsiloxy silicate, and fluorinated silicone resin can be mentioned.

As the absorption promoter, for example, 1-dodecylazacycloheptan-2-one, pyrothiodecane, oleyl alcohol, lauric acid, oleic acid, sodium lauryl sulfate, d-limonene, 1-menthol, 2-pyrrolidone, 1-methyl-2-pyrrolidone, N,N-dimethylformamide, N,N-dimethylacetamide, dimethyl sulfoxide, decylmethyl sulfoxide, N-lauroylsarcosine, isopropyl myristate, isopropyl palmitate, fumaric acid, maleic acid, myristyl lactate, cetyl lactate, polyoxyethylene oleyl ether, lauric acid diethanolamide, polyhydric alcohols, glycerol, propylene glycol, diethanolamine, triisopropanolamine, and triethanolamine can be mentioned. Two or more of these may be used in combination. Preferred is isopropyl myristate.

As the base matrix of the adhesive preparation, for example, acrylic adhesive, silicone adhesive, and rubber adhesive can be mentioned, and appropriate one may be selected from these and used. The matrix may be carried on a support generally used for a preparation to be adhered to the skin, such as tape preparation, patch, cataplasm, plaster and the like, or other support made from a material free of inconvenience for use in the present invention.

As the acrylic adhesive, for example, acrylic acid-octyl acrylate copolymer, acrylate-vinyl acetate copolymer, 2-ethylhexyl acrylate-vinylpyrrolidone copolymer, and methacrylic acid-butyl acrylate copolymer can be mentioned.

As the silicone adhesive, for example, polymethylphenylsiloxane copolymer, and acrylic acid-dimethylsiloxane copolymer can be mentioned.

As the rubber adhesive, for example, one obtained by adding, where necessary, tackifying resin, softener and the like to styrene-isoprene-styrene block copolymer, natural rubber, polyisobutylene, polybutene, and ethylene-vinyl acetate copolymer (EVA) can be mentioned.

As the moisturizer, for example, glycerol, polyethylene glycol, sorbitol, maltitol, propylene glycol, 1,3-butanediol, and reducing maltose starch syrup can be mentioned.

The percutaneous absorption type ophthalmic preparation comprising a muscarinic receptor agonist of the present invention can be manufactured by a conventional method. In the case of an ointment, for example, a muscarinic receptor agonist and ointment base, and, where necessary, a solvent, a medicinal oil, a surfactant, a thickener, a resin, an absorption promoter, and a moisturizer are added and thoroughly mixed to give the ointment. In the case of a gel, a solvent is added to a gel base, the mixture is neutralized with a pH adjusting agent and, where necessary, a solvent, a medicinal oil, a surfactant, a thickener, a resin, an absorption promoter, and a moisturizer are admixed therewith, a muscarinic receptor agonist is added thereto and the mixture is kneaded well to give the gel. In the case of an adhesive preparation (cataplasm, patch, tape, plaster), a base matrix and/or a thickener and, where necessary, a solvent, a medicinal oil, a surfactant, a resin, an absorption promoter, and a moisturizer are added to a muscarinic receptor agonist and the mixture is thoroughly mixed. The plaster is flatted on a support such as a non-woven fabric, a woven fabric, a plastic film (including sheet) or a composite film thereof, and a release liner is applied thereon. Alternatively, the plaster is flatted on a release liner, and the liner is press-adhered onto the aforementioned support. The aforementioned support preferably has flexibility that allows adhesion to the skin surface of the eyelid. While the thickness is appropriately determined according to the dosage form, it is preferably set within the range of 10-3000 µm in consideration of the strength of preparation, uncomfortable feeling and adhesiveness during adhesion. The percutaneous absorption type ophthalmic preparation comprising a muscarinic receptor agonist of the present invention may contain, besides the above-mentioned components, stabilizer, antioxidant, preservative, crosslinking agent, pH adjusting agent, and UV absorber, within the range the effect of the present invention is not impaired.

The muscarinic receptor agonist is generally used in a proportion of 0.1-60 parts by weight, preferably 0.2-20 parts by weight, relative to 100 parts by weight of the base.

Preferably, the content of the muscarinic receptor agonist in the percutaneous absorption type ophthalmic preparation of the present invention is generally 0.1-40 wt%, more preferably 1-30 wt%, particularly preferably 5-30 wt%. When the percutaneous absorption type ophthalmic preparation of the present invention is used as an adhesive preparation, the content of the muscarinic receptor agonist is within the range of preferably 1-40 wt%, more preferably 5-30 wt%, particularly preferably is 15-30 wt%. When it is used as an ointment or gel, the content of the muscarinic receptor agonist is within the range of preferably 0.1-40 wt%, more preferably 1-30 wt%, particularly preferably 5-30 wt%.

The content of the absorption promoter in the percutaneous absorption type ophthalmic preparation of the present invention is generally 1-60 wt%, preferably 5-50 wt%, particularly preferably 10-40 wt%. When the percutaneous absorption type ophthalmic preparation of the present invention is used as an adhesive preparation, the content of the absorption promoter is within the range of preferably 5-60 wt%, more preferably 10-50 wt%, particularly preferably is 20-40 wt%. When it is used as an ointment or gel, the content of the absorption promoter is within the range of preferably 1-60 wt%, more preferably 5-50 wt%, particularly preferably 10-40 wt%.

The mixing ratio of the muscarinic receptor agonist and the absorption promoter in the percutaneous absorption type ophthalmic preparation of the present invention is generally within the range of 1-20 parts by weight of the absorption promoter relative to 1 part by weight of the muscarinic receptor agonist, preferably 1-10 parts by weight of the absorption promoter relative to 1 part by weight of the muscarinic receptor agonist, more preferably 1-5 parts by weight of the absorption promoter. When the percutaneous absorption type ophthalmic preparation of the present invention is used as an adhesive preparation, the mixing ratio of the muscarinic receptor agonist and the absorption promoter is within the range of preferably 1-20 parts by weight of the absorption promoter relative to 1 part by weight of the muscarinic receptor agonist, more preferably 1.5-10 parts by weight of the absorption promoter relative to 1 part by weight of the muscarinic receptor agonist, particularly preferably 2-10 parts by weight of the absorption promoter relative to 1 part by weight of the muscarinic receptor agonist. When it is used as an ointment or gel, the mixing ratio of the muscarinic receptor agonist and the absorption promoter is within the range of preferably 1-20 parts by weight of the absorption promoter relative to 1 part by weight of the muscarinic receptor agonist, more preferably 1-10 parts by weight of the absorption promoter relative to 1 part by weight of the muscarinic receptor agonist, particularly preferably 1-5 parts by weight of the absorption promoter relative to 1 part by weight of the muscarinic receptor agonist.

The percutaneous absorption type ophthalmic preparation of the present invention may contain pharmaceutical components other than the muscarinic receptor agonist, such as steroidal or non-steroidal anti-inflammatory agents, anti-bacterial agents, anti-allergic agents, anti-histamine agents, antiviral agents, vasoconstrictors, therapeutic agents for cataract, therapeutic agents for glaucoma, and mydriatic agents, as long as the objects of the present invention are not impaired.

In the percutaneous absorption type ophthalmic preparation and pharmaceutical composition of the present invention, the dose of the muscarinic receptor agonist varies depending on the pathology, age, administration mode of patients and the like. The daily dose for an adult is generally 0.01 mg-10 g/day, preferably 0.1 mg-1 g/day, more preferably 1 mg-0.2 g/day, which is administered in 1-5 portions as necessary.

Since the percutaneous absorption type ophthalmic preparation of the present invention can promote lacrimal fluid secretion in a sustained manner, and side effects such as miosis are fewer and mild as compared to instillations, it is useful as an agent for the prophylaxis or treatment of dry eye, for example, dry eye associated with ophthalmic diseases such as lacrimal fluidepenia, dry eye, Sjogren's syndrome, keratoconjunctivitis sicca, Stevens-Johnson syndrome, blepharitis, meibomitis and the like, and dry eye caused by VDT (Visual Display Terminal) operation, and use of contact lens.

The subject of administration of the percutaneous absorption type ophthalmic preparation and pharmaceutical composition of the present invention is not particularly limited, and they are useful for the treatment of dry eye in various mammals such as human, monkey, mouse, rat, guinea pig, rabbit, swine, dog, horse, and bovine.

### Examples

The present invention is explained in more detail in the following by referring to Examples and Experimental Examples, which are mere working examples and not to be construed as limitative.

### Example 1

| Pilocarpine hydrochloride-containing ointment | |
|---|---|
| Pilocarpine hydrochloride (manufactured by Nacalai Tesque Inc.) | 0.3 g |
| isopropyl myristate | 1.2 g |
| white petrolatum | 1.5 g |
| total amount | 3 g |

According to the above-mentioned formulation, white petrolatum and isopropyl myristate were mixed well, pilocarpine hydrochloride was added to the mixed ointment base and kneaded well to give a pilocarpine hydrochloride-containing ointment.

### Example 2

| Pilocarpine hydrochloride-containing gel | |
|---|---|
| pilocarpine hydrochloride (manufactured by Nacalai Tesque Inc.) | 0.3 g |
| isopropyl.myristate | 1.2 g |
| 2% carboxyvinyl polymer gel | 1. 5 g |
| total amount | 3 g |

Carboxyvinyl polymer (1 g) is added to purified water (49 mL) by small portions and, after sufficient dispersion and swelling, 8N-NaOH (1 mL) is added to neutralize the mixture to give a transparent 2% carboxyvinyl polymer gel base. Isopropyl myristate is added to this gel base (1.5 g) and the mixture is admixed. Pilocarpine hydrochloride is added and the mixture is kneaded well to give a pilocarpine hydrochloride-containing gel.

### Example 3

| Pilocarpine hydrochloride-containing cataplasm | |
|---|---|
| pilocarpine hydrochloride (manufactured by Nacalai Tesque Inc.) | 0.3 g |
| sodium polyacrylate | 0.45 g |
| glycerol | 0.3g |
| peppermint oil | 0.01 g |
| purified water | appropriate amount |
| total amount | 3 g |

Sodium polyacrylate and glycerol are mixed well with purified water to give a hydrous plaster. Furthermore, peppermint oil and pilocarpine hydrochloride are added and the mixture is kneaded well. The plaster mixture is molded by flatting on a support (polyester non-woven fabric etc.) and a release liner is applied to give a pilocarpine hydrochloride-containing cataplasm.

### Example 4

| Pilocarpine hydrochloride-containing tape | |
|---|---|
| pilocarpine hydrochloride (manufactured by Nacalai Tesque Inc.) | 0.3 g |
| isopropyl myristate | 1.2 g |
| acrylic copolymer | 1.485 g |
| polyisocyanate compound | 0.0015 g |
| ethyl acetate | appropriate amount |
| total amount | 3 g |

Ethyl acetate (about 2 mL) is added to pilocarpine hydrochloride and mixed. After ultrasonication in a disposable cup for about 30 sec to dissolve or disperse pilocarpine hydrochloride, isopropyl myristate is added and the mixture is sufficiently mixed. Then, an acrylic copolymer which is an adhesive base and a polyisocyanate compound which is a crosslinking agent are successively added and the mixture is sufficiently mixed. The mixture is degassed, flatted on a release liner with a doctor knife or Baker applicator and left standing until the organic solvent is evaporated. Then a support is placed thereon, which is pressure adhered with a roller, and the mixture is crosslinked in a thermostatic tank at about 40°C for 8-12 hr to give a pilocarpine hydrochloride-containing tape.

### Example 5

| Cevimeline hydrochloride-containing ointment | |
|---|---|
| cevimeline hydrochloride | 0.3 g |
| isopropyl myristate | 1.2 g |
| white petrolatum | 1.5 g |
| total amount | 3 g |

A cevimeline containing ointment is obtained by preparing in the same manner as in Example 1 and using cevimeline hydrochloride instead of pilocarpine hydrochloride of Example 1.

### Example 6

| Cevimeline hydrochloride-containing gel | |
|---|---|
| cevimeline hydrochloride | 0.3 g |
| isopropyl myristate | 1.2 g |
| 2% carboxyvinyl polymer gel | 1.5 g |
| total amount | 3 g |

A cevimeline hydrochloride-containing gel is obtained by preparing in the same manner as in Example 2 and using cevimeline hydrochloride instead of pilocarpine hydrochloride of Example 2.

### Example 7

| Cevimeline hydrochloride-containing cataplasm | |
|---|---|
| cevimeline hydrochloride | 0.3 g |
| sodium polyacrylate | 0.45 g |
| glycerol | 0.3 g |
| peppermint oil | 0.01 g |
| purified water | appropriate amount |
| total amount | 3 g |

A cevimeline hydrochloride-containing cataplasm is obtained by preparing in the same manner as in Example 3 and using cevimeline hydrochloride instead of pilocarpine hydrochloride of Example 3.

### Example 8

| Cevimeline hydrochloride-containing tape | |
|---|---|
| cevimeline hydrochloride | 0.3 g |
| isopropyl myristate | 1.2 g |
| acrylic copolymer | 1.485 g |
| polyisocyanate compound | 0.0015 g |
| ethyl acetate | appropriate amount |
| total amount | 3 g |

A cevimeline hydrochloride-containing tape is obtained by preparing in the same manner as in Example 4 and using cevimeline hydrochloride instead of pilocarpine hydrochloride of Example 4.

### Example 9

| Carbachol-containing ointment | |
|---|---|
| carbachol | 0.3 g |
| isopropyl myristate | 1.2 g |
| white petrolatum | 1.5 g |
| total amount | 3 g |

A carbachol-containing ointment is obtained by preparing in the same manner as in Example 1 and using carbachol instead of pilocarpine hydrochloride of Example 1.

### Example 10

| Carbachol-containing gel | |
|---|---|
| carbachol | 0.3 g |
| isopropyl myristate | 1.2 g |
| 2% carboxyvinyl polymer gel | 1.5 g |
| total amount | 3 g |

A carbachol-containing gel is obtained by preparing in the same manner as in Example 2 and using carbachol instead of pilocarpine hydrochloride of Example 2.

### Example 11

| Carbachol-containing cataplasm | |
|---|---|
| carbachol | 0.3 g |
| sodium polyacrylate | 0.45 g |
| glycerol | 0.3 g |
| peppermint oil | 0.01 g |
| purified water | appropriate amount |
| total amount | 3 g |

A carbachol-containing cataplasm is obtained by preparing in the same manner as in Example 3 and using carbachol instead of pilocarpine hydrochloride of Example 3.

### Example 12

| Carbachol-containing tape | |
|---|---|
| carbachol | 0.3 g |
| isopropyl myristate | 1.2 g |
| acrylic copolymer | 1.485 g |
| polyisocyanate compound | 0.0015 g |
| ethyl acetate | appropriate amount |
| total amount | 3 g |

A carbachol-containing tape is obtained by preparing in the same manner as in Example 4 and using carbachol instead of pilocarpine hydrochloride of Example 4.

### Example 13

| Pilocarpine hydrochloride-containing adhesive preparation | |
|---|---|
| pilocarpine hydrochloride (manufactured by Nacalai Tesque Inc.) | 0.6 g |
| isopropyl myristate | 1.2 g |
| acrylic copolymer (PE-300) | 1.188 g (solid content) |
| polyisocyanate compound (CK401) | 0.0012 g (solid content) |
| ethyl acetate | appropriate amount |
| total amount | 3 g |

A pilocarpine hydrochloride-containing adhesive preparation was obtained by preparing in the same manner as in Example 4 according to the above-mentioned composition.

### Example 14

| Cevimeline hydrochloride-containing ointment | |
|---|---|
| cevimeline hydrochloride (extracted from Evoxac (trademark) capsule) | 1 g |
| isopropyl myristate | 2 g |
| white petrolatum | 2 g |
| total amount | 5 g |

A cevimeline hydrochloride-containing ointment was obtained by preparing in the same manner as in Example 1 and using cevimeline hydrochloride instead of pilocarpine hydrochloride of Example 1 according to the above-mentioned formulation.

Extraction of cevimeline hydrochloride was performed according to the following method.

### (Extraction and purification of cevimeline hydrochloride)

The contents of Evoxac (trademark) capsule (30 mg) (manufacture No. XAAAE20, 200 capsules (about 42 g), containing cevimeline hydrochloride (30 mg) per capsule) was suspended in purified water (500 mL) and the mixture was vigorously stirred at room temperature. Insoluble material was filtered off, and the mixture was adjusted to pH 10 with 1N aqueous sodium hydroxide solution, and extracted with chloroform (100 mLx4). The organic layer was washed with saturated aqueous sodium hydrogencarbonate solution (100 mLx2) and dried over anhydrous magnesium sulfate. The solvent was evaporated and the residue was dried in vacuo at room temperature for 20 hr to give a pale-yellow oil. The obtained oil was dissolved in diethyl ether, and crystallized by adding equimolar 4N hydrochloric acid/dioxane. The crystals were collected by filtration, washed with diethyl ether, air-dried at room temperature and dried in vacuo at room temperature for 20 hr to give cevimeline hydrochloride (5.78 g). The chemical structure, physical properties and purity of the obtained cevimeline hydrochloride were confirmed by nuclear magnetic resonance spectrum (¹H-NMR) and measurement of melting point.

### Comparative Example 1

| Pilocarpine hydrochloride-containing eye drop | |
|---|---|
| Pilocarpine hydrochloride (manufactured by Nacalai Tesque Inc.) | 1 g |
| sodium dihydrogenphosphate dihydrate | 0.01 g |
| sodium chloride | 0.09 g |
| sodium hydroxide | appropriate amount |
| purified water | appropriate amount |
| total amount | 10 mL (pH 5) |

Sodium dihydrogenphosphate dihydrate and sodium chloride were added to purified water (about 7 mL) for dissolution. Pilocarpine hydrochloride was added to this solution for dissolution and the solution was adjusted to pH 5 with sodium hydroxide. Purified water was added to the total amount (10 mL) to give pilocarpine hydrochloride-containing eye drops.

### Comparative Example 2

| Base adhesive preparation | |
|---|---|
| isopropyl myristate | 1.2 g |
| acrylic copolymer (PE-300) | 1.782 g (solid content) |
| polyisocyanate compound (CK401) | 0.0018 g (solid content) |
| ethyl acetate | appropriate amount |
| total amount | 3 g |

Isopropyl myristate was weighed in a disposable cup. Then, an acrylic copolymer which is an adhesive base and a polyisocyanate compound which is a crosslinking agent were successively added and the mixture was sufficiently mixed. The mixture was degassed, flatted on a release liner with a doctor knife or Baker applicator and left standing until the organic solvent was evaporated. Then a support was placed thereon, which was pressure adhered with a roller, and the mixture was crosslinked in a thermostatic tank at about 40°C for 8-12 hr to give a base adhesive preparation.

### Comparative Example 3

| Pilocarpine hydrochloride-containing eye drop | |
|---|---|
| pilocarpine hydrochloride (manufactured by Nacalai Tesque Inc.) | 2 g |
| sodium dihydrogenphosphate dihydrate | 0.01 g |
| sodium chloride | 0.09 g |
| sodium hydroxide | appropriate amount |
| purified water | appropriate amount |
| total amount | 10 mL (pH 5) |

Pilocarpine hydrochloride-containing eye drops were obtained by preparing in the same manner as in Comparative Example 1 according to the above-mentioned formulation.

### Comparative Example 4

| Cevimeline hydrochloride-containing eye drop | |
|---|---|
| cevimeline hydrochloride (extracted from Evoxac (trademark) capsule) | 2 g |
| sodium dihydrogenphosphate dihydrate | 0.01 g |
| sodium chloride | 0.09 g |
| sodium hydroxide | appropriate amount |
| purified water | appropriate amount |
| total amount | 10 mL (pH 6) |

Cevimeline hydrochloride-containing eye drops were obtained by preparing in the same manner as in Comparative Example 1 and using cevimeline hydrochloride instead of pilocarpine hydrochloride of Comparative Example 1 according to the above-mentioned formulation.

### Experimental Example 1

### Lacrimal fluid secretion test (Schirmer test)

### (Test method)

For the test, rabbits (Japanese white rabbit, male, 9-10-week-old, 2.0-2.8 kg, purchased from KITAYAMA LABES Co., Ltd.) were used. A depilatory treatment was performed in advance for application of ointment. Systemic anesthesia was applied with ketamine/xylazine, and an area around the rabbit eyelid was shaved carefully with a hair clipper and a shaver so that the skin would not be hurt. The pilocarpine hydrochloride-containing ointment prepared in Example 1 was applied by about 7.4 mg (area of about 2 cm × about 4 cm = about 8 cm²) to the skin in a tape stripped area of the upper eyelid and the lower eyelid of one eye of the rabbit, and the opposite eye was not treated. The amount of lacrimal fluid was measured with the lapse of time using Schirmer paper (Whatman No. 41 filter paper was used, manufactured by SHOWA YAKUHIN KAKO Co., Ltd) before the application and after the application. At 5 min before lacrimal fluid measurement, 0.4% oxybuprocaine hydrochloride (Benoxil (trademark) eye drops, 0.4%, manufactured by Santen Pharmaceutical Co., Ltd.) (10 µL) were instilled for topical anesthesia, lacrimal fluid in the lower eyelid conjunctival sac was wiped with filter paper, and the amount of lacrimal fluid secretion was measured for 1 min using Schirmer paper (10 min after application: n=1; 1 hr later: n=2; 2-6 hr later: n=3).

The pilocarpine hydrochloride-containing eye drops prepared in Comparative Example 1, were instilled into one eye (50 µL) and a similar operation was performed (10 min-2 hr after instillation: n=8).

With the amount of lacrimal fluid secretion before application and instillation as the standard (0 mm/min), increase in the amount of lacrimal fluid secretion after application and instillation was measured.

### (Test results)

The results are shown in Fig. 1.

As shown in Fig. 1, the Example 1 administration group showed a large amount of lacrimal fluid secretion and sustained effect as well. In contrast, the Comparative Example 1 administration group showed less amount of lacrimal fluid secretion as compared to the Example 1 administration group, in which a percutaneous absorption type ophthalmic preparation of the present invention was used.

From the above, it is appreciated that the percutaneous absorption type ophthalmic preparation of the present invention is superior in the promotion of lacrimal fluid secretion to eye drops directly administered to the eye, and shows a sustained effect.

### Experimental Example 2

### Effect on pupil diameter

### (Test method)

The pupil diameter was measured simultaneously with Experimental Example 1. Using the pupil diameter before application and instillation as the standard (0 mm), changes in the pupil diameter after application and instillation were measured. For the measurement, electronic digital caliper (MAX-CAL, manufactured by Nippon Sokutei, Japan) was used.

### (Test results)

The results are shown in Fig. 2.

As shown in Fig. 2, mild miosis was observed in the Example 1 administration group, which was relieved rather quickly. In contrast, severe miosis was observed in the Comparative Example 1 administration group.

Therefrom it is clear that the percutaneous absorption type ophthalmic preparation of the present invention causes lower levels of miosis and side effects associated therewith, such as aphose and the like, than eye drops for direct administration to the eye.

It is clear from the results of Experimental Examples 1 and 2 that the percutaneous absorption type ophthalmic preparation of the present invention is a highly safe and superior preparation, wherein the lacrimal fluid secretion by the muscarinic receptor agonist contained in the preparation is persistently promoted by administration to the skin surface of the eyelid, and the side effects such as miosis and the like are fewer.

### Experimental Example 3

### Lacrimal fluid secretion test (Schirmer test)

### (Test method)

For the test, rabbits (Japanese white rabbit, male, 2.3-3.0 kg, purchased from Fukusaki Rabbit Warren) were used. A depilatory treatment was performed in advance for adhesion of a percutaneous absorption preparation. Systemic anesthesia was applied with ketamine/xylazine, and an area around the rabbit eyelid and the back were shaved carefully with a hair clipper and a shaver so that the skin would not be hurt. The pilocarpine hydrochloride-containing adhesive preparation prepared in Example 13 (about 8 cm², about 2 cmxabout 4 cm, total of about 16 cm²) was applied to the skin of each of the upper eyelid and the lower eyelid, from which stratum corneum had been removed by tape stripping, of one eye of the rabbit, and the opposite eye was not treated. The stratum corneum was also removed from the back skin by tape stripping, and the pilocarpine hydrochloride-containing adhesive preparation prepared in Example 13 was adhered by about 16 cm² (about 4 cm×about 4 cm). As a control group, the base adhesive preparation prepared in Comparative Example 2 was adhered to the skin of the upper and lower eyelids, in the same manner as with the pilocarpine hydrochloride-containing adhesive preparation of Example 13. The amount of lacrimal fluid was measured with the lapse of time using Schirmer paper (Whatman No. 41 filter paper was used, manufactured by SHOWA YAKUHIN KAKO Co., Ltd) before the adhesion and after the adhesion. At 5 min before lacrimal fluid measurement, 0.4% oxybuprocaine hydrochloride (Benoxil (trademark) eye drops, 0.4%, manufactured by Santen Pharmaceutical Co., Ltd.) (10 µL) were instilled for topical anesthesia, lacrimal fluid in the lower eyelid conjunctival sac was wiped with filter paper, and the amount of lacrimal fluid secretion was measured for 1 min using Schirmer paper, for the eye (n=3) of the pilocarpine hydrochloride-containing adhesive preparation (Example 13) upper and lower eyelid skin administration group, the opposite eye thereof (non-adhesion, n=3), both eyes (n=6) of the back administration group and the eye (n=3) of the base adhesive preparation (Comparative Example 2) upper and lower eyelid skin administration group.

The pilocarpine hydrochloride-containing, eye drops prepared in Comparative Example 3 were also instilled to one eye by 50 µL̅, and a similar operation was performed (n=3).

Using the lacrimal fluid secretion before administration as the standard (0 mm/min), increase in the amount of lacrimal fluid secretion after the administration was measured.

### (Test results)

The results are shown in Fig. 3.

As shown in Fig. 3, sustained lacrimal fluid secretion was observed in the eye of the Example 13 upper and lower eyelids adhesive administration group, as compared to the Comparative Example 3 instillation administration group. In the Example 13 upper and lower eyelids adhesive administration group, the amount of lacrimal fluid secretion was greater than that of the back administration group. In the Example 13 upper and lower eyelids adhesive administration group, the amount of lacrimal fluid secretion was greater than that of the opposite eye (non-adhesion).

It is clear from the above that the percutaneous absorption type ophthalmic preparation of the present invention is more effective for the promotion of sustained lacrimal fluid secretion than eye drops for direct administration to the eye. It is also clear that administration to the skin surface of the eyelid is superior to the administration to other regions in the lacrimal fluid secretion promoting action. This is considered to be the result of promoting effect of lacrimal fluid secretion afforded by administration to the skin surface of the eyelid, which induces transfer of the muscarinic receptor agonist directly from the eyelid skin to the eye topical tissue rather than to the eye topical tissue via systemic circulation.

### Experimental Example 4

### Effect on pupil diameter

### (Test method)

The pupil diameter was measured simultaneously with Experimental Example 3. Using the diameter size before application as the standard (0 mm), changes in the pupil diameter after application were measured. For the measurement, electronic digital caliper (MAX-CAL, manufactured by Nippon Sokutei, Japan) was used.

Using the amount of lacrimal fluid secretion before administration as the standard (0 mm/min), increase in the amount of lacrimal fluid secretion was measured.

### (Test results)

The results are shown in Fig. 4.

As shown in Fig. 4, miosis was scarcely observed in the Example 13 upper and lower eyelid adhesive administration group. In contrast, severe miosis was observed in the eye of the Comparative Example 3 eye drop administration group.

Therefrom it is clear that the percutaneous absorption type ophthalmic preparation of the present invention causes lower levels of miosis and side effects associated therewith, such as aphose and the like, than eye drops for direct administration to the eye.

### Experimental Example 5

### Lacrimal fluid secretion test (Schirmer test)

### (Test method)

For the test, rabbits (Japanese white rabbit, male, 2.3-2.7 kg, purchased from Fukusaki Rabbit Warren) were used. A depilatory treatment was performed in advance for application of ointment. Systemic anesthesia was applied with ketamine/xylazine, and an area around the rabbit eyelid was shaved carefully with a hair clipper and a shaver so that the skin would not be hurt. The cevimeline hydrochloride-containing ointment prepared in Example 14 was applied by about 7-8 mg (area of about 2 cm × about 4 cm = about 8 cm²) to the skin in a tape stripped area of the upper eyelid and the lower eyelid of one eye of each rabbit, and the opposite eye was not treated. The amount of lacrimal fluid was measured with the lapse of time using Schirmer paper (Whatman No. 41 filter paper was used, manufactured by SHOWA YAKUHIN KAKO Co., Ltd) before the application and 1 hr after the application. At 5 min before lacrimal fluid measurement, 0.4% oxybuprocaine hydrochloride (Benoxil (trademark) eye drops, 0.4%, manufactured by Santen Pharmaceutical Co., Ltd.) (10 µL) were instilled for topical anesthesia, lacrimal fluid in the lower eyelid conjunctival sac was wiped with filter paper, and the amount of lacrimal fluid secretion was measured for 1 min using Schirmer paper.

The cevimeline hydrochloride-containing eye drops prepared in Comparative Example 4 were also instilled to one eye by 50 µL, and a similar operation was performed (n=3).

### (Test results)

The results are shown in Table 1.

**Table 1. Results of lacrimal fluid secretion test (Schirmer test)**

| group | Schirmer Score [mm/min] 1 hr later |
|---|---|
| Example 14 | 7.00 ± 0.50 |
| Comparative Example 4 | 4.50 ± 1.04 |

Each value shows mean±standard error.

As shown in Table 1, a large amount of lacrimal fluid secretion was observed in the Example 14 administration group as compared to Comparative Example 4 administration group. It is clear therefrom that the percutaneous absorption type ophthalmic preparation of the present invention is superior to eye drops for direct administration to the eye in lacrimal fluid secretion promoting action.

From the results of Experimental Examples 1-5 above, it is clear that the percutaneous absorption type ophthalmic preparation of the present invention is a highly safe and superior preparation, wherein the lacrimal fluid secretion by the muscarinic receptor agonist contained in the preparation is persistently promoted by administration to the skin surface of the eyelid, and the side effects such as miosis and the like are fewer.

### Industrial Applicability

According to the present invention, a percutaneous absorption type ophthalmic preparation containing a muscarinic receptor agonist, which is capable of promoting lacrimal fluid secretion by administration to the skin surface of the eyelid, and a method of promoting lacrimal fluid secretion, which comprises administering a percutaneous absorption type ophthalmic preparation containing a muscarinic receptor agonist to the skin surface of the eyelid can be provided. Moreover, the percutaneous absorption type ophthalmic preparation of the present invention is a highly safe preparation associated with few side effects such as miosis and the like observed during use of a preparation for direct administration to the eye such as eye drops and the like.

Moreover, the percutaneous absorption type ophthalmic preparation of the present invention to be administered to the skin surface of the eyelid is superior to administration to regions other than the skin surface of the eyelid in the promotion of sustained lacrimal fluid secretion, which is attributable to the transfer of the muscarinic receptor agonist directly from the eyelid skin to the eye topical tissue rather than to the eye topical tissue via systemic circulation.

The percutaneous absorption type ophthalmic preparation of the present invention can persistently promote lacrimal fluid secretion, and is associated with fewer side effects such as miosis. Therefore, it is useful as an agent for the prophylaxis or treatment of dry eye, for example, dry eye associated with ophthalmic diseases such as lacrimal fluidepenia, dry eye, Sjogren's syndrome, keratoconjunctivitis sicca, Stevens-Johnson syndrome, blepharitis, meibomitis, and dry eye caused by VDT (Visual Display Terminal) operation, use of contact lens.

## Claims

1. A muscarinic receptor agonist to be administered to the skin surface of the eyelid for use in the promotion of lacrimal fluid secretion.

2. The muscarinic receptor agonist of claim 1 and an absorption promoter.

3. The muscarinic receptor agonist of claim 2, wherein the absorption promoter is isopropyl myristate.

4. The muscarinic receptor agonist of any of claims 1 to 3, wherein the muscarinic receptor agonist is pilocarpine or cevimeline, or a pharmaceutically acceptable salt thereof.

5. A percutaneous absorption type ophthalmic preparation comprising a muscarinic receptor agonist, to be administered to the skin surface of the eyelid for use in the promotion of lacrimal fluid secretion.

6. The percutaneous absorption type ophthalmic preparation of claim 5, wherein the content of the muscarinic receptor agonist is 0.1-40 wt%.

7. The percutaneous absorption type ophthalmic preparation of any of claims 5 or 6, which further comprises an absorption promoter.

8. The percutaneous absorption type ophthalmic preparation of claim 7, wherein the content of the absorption promoter is 1-60 wt%.

9. The percutaneous absorption type ophthalmic preparation of claim 7 or 8, wherein the absorption promoter is isopropyl myristate.

10. The percutaneous absorption type ophthalmic preparation of any of claims 5 to 9, wherein the muscarinic receptor agonist is pilocarpine or cevimeline, or a pharmaceutically acceptable salt thereof.

11. The percutaneous absorption type ophthalmic preparation of any of claims 5 to 10, which is an adhesive agent.

12. The percutaneous absorption type ophthalmic preparation of any of claims 5 to 10, which is an ointment.

13. The percutaneous absorption type ophthalmic preparation of any of claims 5 to 10, which is a gel.

## Patentansprüche

1. Muskarinrezeptor-Agonist zur Verabreichung an die Hautoberfläche des Augenlides zur Verwendung bei der Förderung der Sekretion von Tränenflüssigkeit.

2. Muskarinrezeptor-Agonist gemäß Anspruch 1 und ein Resorptionsbeschleuniger.

3. Muskarinrezeptor-Agonist gemäß Anspruch 2, wobei es sich bei dem Resorptionsbeschleuniger um Isopropylmyristat handelt.

4. Muskarinrezeptor-Agonist gemäß einem der Ansprüche 1 bis 3, wobei es sich bei dem Muskarinrezeptor-Agonisten um Pilocarpin oder Cevimelin oder ein pharmazeutisch annehmbares Salz davon handelt.

5. Ophthalmisches Präparat zur perkutanen Resorption, das einen Muskarinrezeptor-Agonisten umfasst, zur Verabreichung an die Hautoberfläche des Augenlides zur Verwendung bei der Förderung der Sekretion von Tränenflüssigkeit.

6. Ophthalmisches Präparat zur perkutanen Resorption gemäß Anspruch 5, wobei der Gehalt des Muskarinrezeptor-Agonisten 0,1-40 Gew.-% beträgt.

7. Ophthalmisches Präparat zur perkutanen Resorption gemäß einem der Ansprüche 5 oder 6, das weiterhin einen Resorptionsbeschleuniger umfasst.

8. Ophthalmisches Präparat zur perkutanen Resorption gemäß Anspruch 7, wobei der Gehalt des Resorptionsbeschleunigers 1-60 Gew.-% beträgt.

9. Ophthalmisches Präparat zur perkutanen Resorption gemäß Anspruch 7 oder 8, wobei es sich bei dem Resorptionsbeschleuniger um Isopropylmyristat handelt.

10. Ophthalmisches Präparat zur perkutanen Resorption gemäß einem der Ansprüche 5 bis 9, wobei es sich bei dem Muskarinrezeptor-Agonisten um Pilocarpin oder Cevimelin oder ein pharmazeutisch annehmbares Salz davon handelt.

11. Ophthalmisches Präparat zur perkutanen Resorption gemäß einem der Ansprüche 5 bis 10, bei dem es sich um ein Klebemittel handelt.

12. Ophthalmisches Präparat zur perkutanen Resorption gemäß einem der Ansprüche 5 bis 10, bei dem es sich um eine Salbe handelt.

13. Ophthalmisches Präparat zur perkutanen Resorption gemäß einem der Ansprüche 5 bis 10, bei dem es sich um ein Gel handelt.

## Revendications

1. Agoniste de récepteur muscarinique à administrer à la surface de la peau de la paupière à utiliser dans la promotion de la sécrétion du liquide lacrymal.

2. Agoniste de récepteur muscarinique selon la revendication 1, et promoteur d'absorption.

3. Agoniste de récepteur muscarinique selon la revendication 2, dans lequel le promoteur d'absorption est le myristate d'isopropyle.

4. Agoniste de récepteur muscarinique selon l'une quelconque des revendications 1 à 3, dans lequel l'agoniste de récepteur muscarinique est la pilocarpine ou la céviméline, ou l'un de leurs sels pharmaceutiquement acceptables.

5. Préparation ophtalmique de type à absorption percutanée comprenant un agoniste de récepteur muscarinique, à administrer à la surface de la peau de la paupière à utiliser dans la promotion de la sécrétion du liquide lacrymal.

6. Préparation ophtalmique de type à absorption percutanée selon la revendication 5, dans laquelle la teneur de l'agoniste de récepteur muscarinique est de 0,1 à 40 % en poids.

7. Préparation ophtalmique de type à absorption percutanée selon l'une quelconque des revendications 5 ou 6, qui comprend en outre un promoteur d'absorption.

8. Préparation ophtalmique de type à absorption percutanée selon la revendication 7, dans laquelle la teneur du promoteur d'absorption est de 1 à 60 en poids.

9. Préparation ophtalmique de type à absorption percutanée selon la revendication 7 ou 8, dans laquelle le promoteur d'absorption est le myristate d'isopropyle.

10. Préparation ophtalmique de type à absorption percutanée selon l'une quelconque des revendications 5 à 9, dans laquelle l'agoniste de récepteur muscarinique est la pilocarpine ou la céviméline, ou l'un de leurs sels pharmaceutiquement acceptables.

11. Préparation ophtalmique de type à absorption percutanée selon l'une quelconque des revendications 5 à 10, qui est un agent adhésif.

12. Préparation ophtalmique de type à absorption percutanée selon l'une quelconque des revendications 5 à 10, qui est une pommade.

13. Préparation ophtalmique de type à absorption percutanée selon l'une quelconque des revendications 5 à 10, qui est un gel.
